# EUROPÄISCHE PATENTSCHRIFT

(11) **EP 0 703 793 B1**
(45) Veröffentlichungstag und Bekanntmachung des Hinweises auf die Patenterteilung: **25.11.1998**
(21) Anmeldenummer: 94918724.9
(22) Anmeldetag: 16.06.1994
(51) Int. Cl.: A61L 2/06

(54) **VERFAHREN ZUR HERSTELLUNG EINER STERILEN BEREITSCHAFTSPACKUNG UND BEHÄLTNIS FÜR EINE SOLCHE BEREITSCHAFTSPACKUNG**
PROCESS FOR PRODUCING STERILE PACKINGS OF READY-FOR-USE ARTICLES AND CONTAINER FOR SUCH PACKINGS
PROCEDE DE FABRICATION D'UN CONDITIONNEMENT STERILE DE PRODUITS PRETS-A-L'EMPLOI ET RECIPIENT POUR CE CONDITIONNEMENT

(30) Priorität: 17.06.1993 DE 4320066
(43) Veröffentlichungstag der Anmeldung: 03.04.1996
(73) Patentinhaber: FARCO-PHARMA GESELLSCHAFT MIT BESCHRÄNKTER HAFTUNG PHARMAZEUTISCHE PRÄPARATE, D-50829 Köln (DE)
(72) Erfinder: WOLF, Erich, Dr., D-51491 Overath (DE)
(74) Vertreter: Clemens, Gerhard, Dr.-Ing.
(86) Internationale Anmeldenummer: DE9400680
(87) Internationale Veröffentlichungsnummer: WO9500180

(56) Entgegenhaltungen:
- EP-A- 0 227 401
- EP-A- 0 322 134
- FR-A- 2 521 906
- US-A- 5 207 983

## Beschreibung

### TECHNISCHES GEBIET

Die Erfindung betrifft ein verfahren zur Herstellung einer Bereitschaftspackung mit zumindest einem eingesiegelten, insbesondere verblisterten, mit einem Arzneimittel gefüllten Behältnis, insbesondere Spritze, Beutel, Flasche oder sonstigegem medizinischen Bedarfsgegenstand, bei dem die Packung, insbesondere Blisterpackung, einen bevorzugt für Wasserdampf durchlässigen Bereich aufweist und im übrigen dicht ist, bei dem für das Behaltnis ein mindestens bei 125°C (Grad Celsius), vorzugsweise bei 130°C, beständiges Material eingesetzt wird und bei dem die versiegelt verschlossene Packung einem Autoklaviervorgang ausgesetzt wird.

Beim Erhitzen von Wasser in einem geschlossenen Druckbehalter wird bei 121°C ein Überdruck von 1 bar erreicht. Der "gespannte" Wasserdampf reicht bei einer Einwirkungsdauer von 15 bis 20 Minuten aus, alle vermehrungsfähigen Mikroorganismen abzutöten.

### STAND DER TECHNIK

Bei einer bekannten Bereitschaftspackung dieser Art werden die Einzelteile der aus Kunststoff bestehenden Spritze zur Sterilisation mit Ethylenoxid begast. Dann wird unter Sterilbedingungen die Spritze zusammengesetzt und mit der vorsterilisierten Füllung unter aseptischen Bedingungen gefüllt und verschlossen. Dann wird die gefüllte Spritze verblistert zu einer versiegelten Packung, die dann zur Sterilisation mit Ethylenoxid begast wird. Das Gas dringt dabei durch Porösitäten der Umhüllung und umspült die Spritze von außen.

Bei diesem Verfahren ist die Herstellung der gefüllten Spritze, da sie unter Sterilbedingungen erfolgt, aufwendig und es müssen auch aufwendige Vorkehrungen getroffen werden, um zu vermeiden, daß Ethylenoxidreste oder Zerfallprodukte aus der Begasung der Spritze und der fertigen Packung in die/der Füllung der Spritze gelangen bzw. verbleiben.

Die US-PS 48 28 797 zeigt die Herstellung eines biologischen Test-Kits, mit dessen Hilfe die Effektivität z.B. einer Ethylenoxidsterilisation überprüft werden kann. Hierbei wird eine offene Kunststoff-Einmalspritze in einem Blister mit einer Tyvek (eingetragenes Warenzeichen)-Folie durch Sterilisation entkeimt. Dabei befindet sich im Inneren der Spritze ein Teststreifen, der sich bei ausreichender Sterilisation, z .B. durch Ethylenoxid, verfärbt.

Die US-PS 50 33 252 offenbart ein Sterilisationsverfahren, bei dem eine mit einer sterilen Salzlösung gefüllte Flasche in einen Blister gegeben wird, welcher anschließend einer Ethylenorydbegasung oder gespanntem Wasserdampf ausgesetzt wird. Hierdurch wird nur die Entkeimung der Flaschenaußenseite erreicht. Dieses Verfahren setzt voraus, daß die Flasche ohne Restluft im Inneren der Flasche vollständig gefüllt sein muß und die Befüllung der Flasche unter aseptischen Bedingungen erfolgen muß. Dabei kommt es regelmäßig zum Überlaufen der Füllung, um zu gewährleisten, daß sich keine Luft im Inneren der Flasche befindet. Daher ist die Flasche selbst und deren Umgebung nach dem Befüllen zu reinigen. In Verbindung mit dem aseptischen Füllvorgang und Verschließvorgang ist dieses Verfahren sehr aufwendig und daher wenig wirtschaftlich.

EP 0 227 401 B1 beschreibt ein Verfahren zur Endsterilisation einer Kunststoff-Spritze samt Inhalt (Flüssigkeit) mittels Dampf und Stützdruck. Dieses verfahren ist für Glas-Fertigspritzen bereits bei Venten & Hoppert,Pharm.Ind., Bd. 40 (1978), S. 665 ff. Punkt 4 eingehend mit Bildern beschrieben.

### DARSTELLUNG DER ERFINDUNG

Erste Aufgabe der Erfindung ist es, die Herstellung einer sterilen Bereitschaftspackung der eingangs genannten Art zu vereinfachen und eine wirtschaftliche und umweltfreundliche Herstellung mit einer Autoklavierung der Endverpackung zu ermöglichen.

Das erfindungsgemäße Verfahren ist durch die Merkmale des Anspruchs 1 gegeben. Vorteilhafte Ausgestaltungen und Weiterbildungen sind Gegenstand der Unteransprüche.

Das erfindungsgemäße Verfahren zeichnet sich demgemäß dadurch aus, daß in einem einzigen Arbeitsgang sowohl die Füllung mindestens 15 Minuten, bevorzugt 20 Minuten, bei mindestens 121°C mittels Dampf-Luftgemisch-Umwälzverfahren sterilisiert wird als auch die Außenoberfläche des Behältnisses als auch die Packung sterilisiert wird.

Erfindungsgemäß ist erkannt worden, daß es besonders wirtschaftlich ist, die Bereitschaftspackung in einem Schritt zu autoklavieren, indem sowohl die Innenseiten der Packung (Blister), die Außenseiten des verschlossenen Behältnisses, als auch das Füllgut (flüssiges, gelförmiges oder pastöses Arzneimittel) in einem einzigen Behandlungsvorgang sterilisiert werden.

Eine besonders bevorzugte Weiterbildung des erfindungsgemäßen Verfahrens zeichnet sich dadurch aus, daß mehrere Bereitschaftspackungen in einer Verpackungseinheit (Faltschachtel) angeordnet werden, welche einen für Wasserdampf durchlässigen Bereich aufweist, und in zumindest einer Bereitschaftspackung, bevorzugt zumindest zwei Bereitschaftspackungen, der gesamten Autoklavenladung die Innentemperatur des flüssigen, gelförmigen oder pastösen Arzneimittels gemessen wird und davon abhängig der Sterilisationszyklus und die Druckdifferenzen zwischen Arzneimittel, Bereitschaftspackung und Autoklavenkammer beim Autoklavierungszyklus geregelt wird.

In besonders vorteilhafter Art und Weise wird darüberhinaus so vorgegangen, daß während des gesamten Sterilisationszyklus der Druck und der Feuchtigkeitsgehalt gemessen werden und in Abhängigkeit der gemessenen Werte der Sterilisationszyklus geregelt wird, wobei der Druck bevorzugt mit einer vorgebbaren Druckänderungsgeschwindigket änderbar ist.

Dadurch daß die Temperatur im Arzneimittel (oder Gut) selbst gemessen wird, kann ein einwandfreier Ablauf des Sterilisationszyklus direkt ohne jeden Zweifel gewährleistet und dokumentiert werden. Ist die gemessene Temperatur zu niedrig, wird über eine Steuereinrichtung eine Heizeinrichtung beaufschlagt, die zusätzlich Wärme (Dampf) direkt oder indirekt in die Autoklavenkammer bringt. Dadurch daß gleichzeitig der Druck und die Feuchtigkeit kontrolliert werden, kann eine Beschädigung der Bereitschaftspackung bzw. der Verpackungseinheit infolge auftretender Druckunterschiede oder aufgrund von sich niederschlagendem Kondenswasser verhindert werden. Bevorzugt ist der Druck hierbei mit einer Geschwindigkeit von +/- 0,1 bar pro 5 Minuten änderbar. Es ist beispielsweise als maximaler Stützdruck beim Abkühlen ein Druck von 3,0 bar vorgesehen. Um einen Druckabbau zu ermöglichen, weist der Autoklav eine Evakuiereinrichtung auf, die entsprechend den gemessenen Werten geregelt wird.

Für die Verpackungseinheit, die mehrere Bereitschaftspackungen enthält, wird als Material in einer bevorzugten Faltschachtel-Ausgestaltung Karton eingesetzt, welcher Karton vorzugsweise zumindest bereichsweise perforiert ausgebildet ist, damit der Wasserdampf ohne großen Widerstand in das Innere der Verpackungseinheit gelangen kann. Ebenso kann beispielsweise für die Verpackungseinheit ein Papiersack eingesetzt werden. Hinsichtlich Wirtschaftlichkeit und der Entsorgung der Bereitschaftspackung ist es vorteilhaft, den für Wasserdampf durchlässigen Bereich als Papierbereich auszubilden. In Verbindung mit der Kartonausbildung der Verpackungseinheit treten somit keine Entsorgungsprobleme auf, da die genannten Materialien problemlos der Wiederverwertung zugeführt werden können.

Die Dampfsterilisation erfolgt bei einem bevorzugten Ausführungsbeispiel an gefüllten, verblisterten und in Zehner-Einheiten in Faltschachteln verpackte Spritzen. Durch die Feuchtigkeitskontrolle des Luft-Wasserdampfgemisches auf einen empirisch ermittelten, optimalen Feuchtigkeitsgehalt im Grenzbereich Dampf/Kondensat wird gewährleistet, daß dadurch eine rasche Wärmeübertragung durch die bevorzugt perforierte Faltschachtel und das Papier der Bereitschaftspackung zur Spritzenaußenseite erreicht wird. Die Außenseite der Spritze muß so aufgeheizt und befeuchtet sein, daß im Füllinhalt (flüssiges, gelförmiges oder pastöses Arzneimittel) bevorzugt über 20 Minuten eine Temperatur von 121°C erreicht wird.

Durch die erfindungsgemäße Optimierung des Dampf-/Kondensatbereichs während des Autoklavierens werden optische Veränderungen (Wasserflecken) sowohl auf dem Sterilpapier des Blisters als auch auf der Faltschachtel vermieden.

Im Gegensatz zu der bei dem bekannten Verfahren eingesetzten Spritze ist die nach der Erfindung eingesetzte Spritze bei 125 ^{o}C beständig und deshalb mit gespanntem Dampf sterilisierbar. Dadurch wird es möglich, die fix und fertig versiegelte Bereitschaftspackung thermisch zu sterilisieren. Während bei der Ethylenoxid-Begasung der Bereitschaftspackung die Sterilisation nur auf das Äußere des Spritzenkörpers wirkt, kann durch die Dampf-Luftgemisch-Sterilisation auch das Innere des Spritzenkörpers (Behältnisses) und dessen Füllung sterilisiert werden. Dadurch aber werden aufwendige Herstellungsmaßnahmen zur Bereitstellung einer steril abgefüllten Spritze vermeidbar und da keinerlei mikrobizide Gase zur Sterilisation eingesetzt werden müssen, stellt sich das Problem mit den Rückständen solcher Gase nicht mehr.

Weiterhin erübrigt sich eine aseptische Abfüllung des flüssigen, gelförmigen oder pastösen Arzneimittels, die bekanntermaßen gegenüber einer Endsterilisation des Arzneimittels im verschlossenen Endbehältnis Risiken mikrobieller Kontamination aufweist.

Sterilisation durch Autoklavieren bei 121 ^{o}C über 20 Minuten führt bekanntlich zu einer hinreichenden Sterilisation. Man muß allerdings durch Bemessung der Sterilisationszeit und auch der angewendeten Temperatur dafür Sorge tragen, daß die Sterilisationsbedingungen - 121 ^{o}C und 20 Minuten Dauer - auch im Innersten der Füllung herrschen. Angesichts des unvermeidbaren Temperaturgefälles erfordert es bei einer Kerntemperatur in der Füllung von 121 ^{o}C eine Temperatur von einigen Grad Celsius darüber für den Spritzenkörper und das ist der Grund, weshalb für den Spritzenkörper, obwohl 121 ^{o}C für die Sterilisation ausreichen, eine um mindestens einige Grad Celsius höhere Temperaturbeständigkeit vorgesehen ist.

Als besonders geeignetes Material für die Spritze wird ein thermostabiler Kunststoff, insbesondere Polypropylen, oder Glas eingesetzt, wobei erfindungsgemäß eine Kolbenstange in der Spritze eingesetzt werden kann, die bereichsweise eine Gummidichteinrichtung aufweist.

Das erfinderische Verfahren zum Herstellen einer Bereitschaftspackung mit Spritze wird vorzugsweise derart ausgeübt, daß eine leere mit einem Ausspritzkonus versehene Spritze mit in Entleerungsstellung vorgeschobenem Kolben bereitgehalten wird, daß in die bereitgehaltene Spritze durch den Ausspritzkonus Füllung dosiert eingedrückt wird und dadurch der Kolben nach rückwärts zurückgedrückt wird, daß der Ausspritzkonus der gefüllten Spritze mit einer Verschlußkappe aus zumindest bei 121 ^{o}C beständigem elastischen Material, vorzugsweise aus temperaturbeständigem Gummi oder Kunststoff, verschlossen wird, daß die gefüllte, verschlossene Spritze in einen Blister eingesetzt wird, daß der Blister verschlossen und versiegelt wird, daß die Bereitschaftspackung oder eine mehrere Bereitschaftspackungen aufweisende Verpackungseinheit im Autoklav, vorzugsweise nach vorheriger Evakuierung, im Wasserdampf auf 121 ^{o}C durchgewärmt und über mindestens 20 Minuten auf 121 ^{o}C durchgewärmt gehalten wird und daß die Bereitschaftspackung dann langsam, vorzugsweise unter Einführung eines Stützdrucks, abgekühlt und dabei dekompressiert und durch ein- oder mehrmalige Evakuierung vollständig entfeuchtet wird.

Eine weitere erfindungsgemäße Verfahrenvariante zeichnet sich dadurch aus, daß eine Spritze ohne Kolbenstange bereitgehalten wird, deren Ausspritzkonus mit einer Verschlußkappe oder einer mit einer Nadelschutzkappe verschlossenen, mit der Spritze fest verbundenen Injektionskanüle oder einem Applikator dicht verschlossen ist, die aus zumindest bei 121 ^{o}C beständigem elastischen Material besteht, in die bereitgehaltene Spritze von der Öffnung für die Kolbenstange her dosiert Füllung eingebracht wird, in die gefüllte Spritze die Kolbenstange dichtend eingeführt wird, die gefüllte, verschlossene Spritze in einen Blister eingesetzt wird, der Blisterverschluß nun versiegelt wird, die Bereitschaftspackung oder eine mehrere Bereitschaftspackungen enthaltende Verpackungseinheit im Autoklav, vorzugsweise nach vorheriger Evakuierung, im Wasserdampf auf 121 ^{o}C durchgewärmt und über mindestens 20 Minuten auf 121 ^{o}C durchgewärmt gehalten wird und die Bereitschaftspackung dann langsam, vorzugsweise unter Einführung eines Stützdrucks, abgekühlt und dabei dekompressiert und durch ein- oder mehrmaliges Evakuieren der Autoklavenkammer vollständig entfeuchtet wird.

In einer bevorzugten Ausgestaltung wird das Behältnis so in die Bereitschaftspackung eingelegt bzw. ist die Wandung der Bereitschaftspackung so an das Behältnis angepaßt, daß eine zeitweise auftretende mögliche Längenausdehnung bzw. Längenverschiebung infolge des Sterilisations- (Erwärmungs-)Vorgangs durch die Wandung des Behältnisses zumindest teilweise unterdrückt wird. So kann insbesondere bei Spritzen das Ausweichen des Spritzenstempels durch die Erwärmung des Füllinhaltes und der Restluftblase verhindert werden, wobei insgesamt die Spritze so in der Bereitschaftspackung, bevorzugt im Blister, fixiert wird, daß kein Füllgut aus der Spritze austreten kann.

Eine zweite Aufgabe der Erfindung besteht darin, eine Spritze so auszugestalten, daß sie zur Ausübung des erfinderischen Verfahrens vorteilhaft geeignet ist und bei der Herstellung, Füllung und Benutzung leicht zu handhaben ist.

Eine entsprechend ausgestaltete Spritze ist dadurch gekennzeichnet, daß ein Spritzenkörper einen kreiszylindrischen, nach hinten auf seiner ganzen Querschnittsfläche offenen und nach vorn in einen Ausspritzkonus mündenden mindestens umhüllten Füllraum aufweist, daß eine langgestreckte Kolbenstange koaxial zum Füllraum angeordnet ist, die mit ihrem hinteren Ende aus dem Füllraum herausragt, an ihrem hinteren Ende eine-Handhabe und an ihrem vorderen Ende einen Kolben aufweist, der längsverschieblich in den Füllraum paßt, daß der Kolben zumindest eine stirnseitig an der Kolbenstange angeordnete, dichtend in den Füllraum passende Kolbenscheibe aufweist, daß eine auf den Ausspritzkonus aufsetzbare Verschlußkappe vorgesehen ist und daß alle Teile aus mindestens bei 121 ^{o}C beständigem Material, vorzugsweise Kunststoff, bestehen. Bevorzugt ist hinter der einen ersten Kolbenscheibe eine zweite, nur führend in den Füllraum passende Kolbenscheibe vorhanden.

Durch die beiden Kolbenscheiben wird eine axiale Führung der Kolbenstange sowohl beim Füllen als auch bei der Benutzung gewährleistet, ohne daß dadurch die Herstellung der Kolbenstange erschwert wird. Im Gegenteil, bekannte, zur Führung axial langgestreckte Kolben, erfordern mehr Kunststoffmaterial als die beiden vorgesehenen Kolbenscheiben.

Das Füllen der zusammengesetzten Spritze erfolgt bei einer erfindungsgemäßen Ausführungsform durch den offenen Ausspritzkonus. Dabei wird die Dosierung erleichtert, wenn, wie vorzugsweise vorgesehen, ein innerer Anschlag am hinteren Ende der den Füllraum umhüllenden Innenwandung vorgesehen ist, der als hinterer Anschlag für die zweite Kolbenscheibe in Füllstellung des Kolbens wirksam ist und dem Fülldruck standhält, mit größerer Kraftanstrengung dagegen durch den Kolben überwindbar ist. Man kann den Fülldruck bei Erreichen der für die Füllung vorgesehenen Dosierung absenken oder von vornherein so niedrig halten, daß er den Anschlag nicht überwindet. Auf diese Weise lassen sich Toleranzen bei der Dosierung der Füllung sehr eng halten.

Der Anschlag kann ein einzelner Vorsprung oder es können auch eine Reihe auf den Umfang verteilt angeordnete Vorsprünge vorgesehen sein, vorzugsweise ist jedoch der Anschlag ein zum Füllraum konzentrischer Wulst.

Bei einer bevorzugten Ausgestaltung der erfindungsgemäßen Spritze weist die Kolbenscheibe eine umlaufende Dichteinrichtung, insbesondere eine O-Ring-Dichteinrichtung aus Gummi, auf, die auch nach dem Autoklavieren eine gute Dichtwirkung dauerhaft gewährleistet. Eine alternative erfindungsgemäße Ausführungsvariante zeichnet sich dadurch aus, daß stirnseitig vor der Kolbenscheibe eine in Kolbenrichtung elastisch verformbare Dichtungseinrichtung, insbesondere ein Gummistopfen, mit zumindest einer umfangsmäßig umlaufenden Dichtlamelle vorhanden ist. Durch die elastische Verformbarkeit kann der sich im Inneren aufbauende Druck während des Sterilisationsvorgangs teilweise aufgefangen werden, insbesondere bei Vorhandensein von Luftblasen.

Vorteilhafte Ausgestaltungen und Weiterbildungen sind Gegenstand weiterer Unteransprüche.

Die Merkmale der Ansprüche können in beliebiger Weise miteinander kombiniert werden, insoweit sie sich nicht offensichtlich gegenseitig ausschließen.

Weitere Ausführungsformen und Vorteile der Erfindung ergeben sich durch die nachstehend angegebenen Ausführungsbeispiele.

Die Erfindung sowie vorteilhafte Ausführungsformen und Weiterbildungen derselben werden im folgenden anhand der in der Zeichnung dargestellten Beispiele näher beschrieben und erläutert. Die der Beschreibung und der Zeichnung zu entnehmenden Merkmale können einzeln für sich oder zu mehreren in beliebiger Kombination angewandt werden.

### KURZE BESCHREIBUNG DER ZEICHNUNG

In der Zeichnung zeigt:
- Fig. 1: im Längsschnitt eine fertige Bereitschaftspackung,
- Fig. 2: den Schnitt II aus Figur 1,
- Fig. 3: den Ausschnitt III aus Figur 1 vergrößert,
- Fig. 4, 5: schematischen Detailschnitt einer Spritze mit Kolbenstange und Dichtmitteln und
- Fig. 6: schematischen Schnitt durch einen Autoklaven mit einer mehrere Bereitschaftspackungen enthaltenden Verpackungseinheit mit Temperatur-, Druck- und Feuchtigkeitsmessung während des Autoklavierens.

### WEGE ZUM AUSFÜHREN DER ERFINDUNG

In der Zeichnung ist mit 1 allgemein die Bereitschaftspackung bezeichnet. Diese besteht aus einer mit der Füllung 2 gefüllten Spritze 3 und einer versiegelt verschlossenen Packung 4 für diese Spritze. Die Spritze 3 besteht aus dem Spritzenkörper 5, der Kolbenstange 6 und einer Verschlußkappe 7.

Der Spritzenkörper 5 ist kreiszylindrisch langgestreckt und weist hinten eine sich über seine ganze innere Querschnittsfläche erstreckende Öffnung 10 auf. An seinem vorderen Ende mündet der Spritzenkörper in einen Ausspritzkanalkörper 8 mit einem Ausspritzkanal 9, der durch die aufsteckbare Verschlußkappe 7 verschließbar ist. Der Spritzenkörper umhüllt den mit der fließfähigen Füllung 2, vorzugsweise einem flüssigen, gelförmigen oder pastösen Arzneimittel, ausgefüllten Füllraum 11, und zwar mindestens durchscheinend, vorzugsweise sogar durchsichtig. Am rückwärtigen Ende des Spritzenkörpers ist eine als Handhabe dienende ovale Scheibe 22 koaxial zur Spritzenachse 14 angeordnet.

Die Kolbenstange 6 besteht aus zwei gekreuzt angeordneten flachen Leisten 12, 13. Sie ragt mit ihrem hinteren Ende aus dem Füllraum 11 heraus und weist dort eine konzentrisch zur Spritzenachse 14 aufgesetzte Druckscheibe 15 als Handhabe auf. An ihrem vorderen Ende weist sie einen in den Füllraum gesteckten Kolben 16 auf. Dieser Kolben 16 besteht aus einer stirnseitig an der Kolbenstange koaxial zur Spritzenachse 14 angeordneten ersten Kolbenscheibe 17 und einer mit Abstand dahinter koaxial zur Spritzenachse 14 angeordneten zweiten Kolbenscheibe 18.

Die erste Kolbenscheibe 17 paßt dichtend in den Füllraum 11, während die zweite Kolbenscheibe nur führend in den Füllraum 11 paßt. Die beiden Kolbenscheiben 17 und 18 sind entsprechend dem Querschnitt des Füllraums 11 kreisrund. Die zweite Kolbenscheibe 18 kann an ihrem Umfang auch Ausnehmungen aufweisen, wesentlich ist nur, daß sie formbedingt hinreichende Führung bietet. Die Füllung 2 endet an der ersten Kolbenscheibe 17.

Mit 19 ist ein als innerer Anschlag für die zweite Kolbenscheibe 18 dienender, konzentrischer in den Füllraum 11 hineinragender Wulst bezeichnet, der sich am hinteren Ende an der Innenwand des Spritzenkörpers erstreckt. Dieser Wulst bildet für die zweite Kolbenscheibe 18, und zwar in Füllstellung des Kolbens 16, einen hinteren Anschlag, der einem für die Füllung der Spritze durch den Ausspritzkanal 9 ausreichenden Fülldruck standhält, aber mit größerer Kraftanstrengung von beiden Kolbenscheiben überwindbar ist.

Der Spritzenkörper 5 besteht aus Kunststoff oder Glas. Die Verschlußkappe 7 besteht aus Gummi. Die Kolbenstange 6 besteht aus Kunststoff, gegebenenfalls mit einem Stopfen oder O-Ring aus Gummi. Die Einzelteile sind sämtlich bei 125°C, vorzugsweise bei 130°C, temperaturbeständig.

Die Packung 4 - auch Verblisterung genannt - besteht aus einer flachen Sohle 20 und einer Haube 21. Die Sohle 20 besteht aus stabilem Papier, das für Wasserdampfdiffusion durchlässig, im übrigen aber dicht ist, die Haube 21 besteht aus durchsichtigem Kunststoff. Haube und Sohle sind mindestens bei 121°C, bevorzugt 125 ^{o}C, beständig. Die Packung ist hinreichend steif, so daß mehrere Bereitschaftspeckungen übereinander gestapelt werden können, ohne daß die Packung eindrückt. Die Haube 21 ist mit der Sohle 20 dicht verschweißt. Die Haube 21 ist dampfdicht.

Die Haube 21 ist dabei so ausgebildet, daß ihre stirnseitigen Wandbereiche 21.1 bzw. 21.2 bei eingesetzter Spritze an der Stirnseite der Druckscheibe 15 und der Stirnseite der Verschlußkappe 7 anliegen. Dadurch wird ein Ausweichen der Kolbenstange während des Sterilisiervorgangs unterbunden. Durch die Erhitzung der Füllung 2 und/oder durch im Inneren des Spritzenkörpers 5 vorhandene Luftblasen kommt es infolge Erwärmung zu einer Längenausdehnung bzw. zum Aufbau eines Innendrucks. Dadurch könnten die Kolbenstange und die Verschlußkappe von dem Spritzenkörper geschoben werden, so daß unerwünschterweise die Füllung austritt. Dies wird erfindungsgemäß dadurch verhindert, daß die Längenausdehnung dadurch zumindest teilweise verhindert wird, indem die Packung 1,4 selbst die Beanspruchung infolge Längenausdehnung der Spritze 3 aufnimmt.

Darüberhinaus ist die Verschlußkappe 7 um ein vorgebbares Maß längsverschieblich auf dem zylindrisch ausgebildeten Ausspritzkanalkörper 8 angeordnet, ohne daß bei einer Verschiebung der Verschlußkappe 7 um diesen vorgegebenen Weg die Füllung aus dem Ausspritzkanal 9 austreten kann. Durch diese Verschiebbarkeit wird ebenfalls einem sich aufbauenden Druck im Inneren des Spritzenkörpers 5 entgegengewirkt.

Zur Herstellung der Bereitschaftspackung wird wie folgt vorgegangen.

Die sauber hergestellte Spritze wird mit nach vorn in den Spritzenkörper hineingeschobenem Kolben und abgezogener Verschlußkappe 7 bereitgehalten. Es wird nun flüssiges, gelförmiges oder pastöses Füllgut, insbesondere Arzneimittel oder Diagnostika, über den Ausspritzkanal 9 eingedrückt und dadurch die Kolbenstange 6 nach hinten verschoben. Wenn der dabei aufgewendete Fülldruck so niedrig ist, daß der durch den Wulst 19 gebildete Anschlag nicht überwunden werden kann, kann dieser Fülldruck bis zum Schluß beibehalten bleiben, andernfalls wird er kurz vor Erreichen der vollständigen Füllung so weit abgesenkt, daß der Anschlag nicht überwunden werden kann. Sobald die zweite Kolbenscheibe 18 an den Wulst 19 anschlägt, ist der Füllvorgang beendet und die Verschlußkappe 7 wird aufgesetzt.

Anschließend wird die gefüllte Spritze in die vorbereitet hergestellte Haube 21 gelegt, dann wird die Sohle 20 aufgelegt und mit der Haube auf dem ganzen Umfang verschweißt (Verblisterung) . Ein oder mehrere Blister werden in Faltschachteln verpackt und in einem Autoklav wie folgt sterilisiert.

Der Autoklav wird zunächst evakuiert bis auf 40 Torr. Dann wird die Autoklavenkammer durch Dampfzufur auf 125 ^{o}C erhitzt. Der dazu erforderliche Druck wird so langsam aufgebaut, daß durch Wasserdampfdiffusion in dem Zwischenraum zwischen Spritze und Packung im Inneren der Bereitschaftspackung der Druck so weit folgen kann, daß die Bereitschaftspackung nicht eingedrückt wird. Die Temperatur von 125 ^{o}C wird so lange gehalten bis die Sterilisation des Füllguts im Behältnis abgeschlossen ist. Die Temperatur wird durch Meßfühler im Füllgut gemessen und registriert, wobei in einer Autoklavenladung immer mindestens zwei verschlossene Behältnisse mit einem Temperaturfühler versehen sind. Nach der Sterilisation wird das sterilisierte Gut in der Kammer indirekt abgekühlt. Zur Entfernung unzulässiger Restfeuchte wird die Autoklavenkammer ein- oder mehrmals während und nach der Abkühlung evakuiert . Anschließend wird abgekühlt auf Außentemperatur und der Autoklav belüftet und geöffnet. Die Abkühlung erfolgt unter gleichzeitiger Einführung eines mit Luft erzeugten Stützdruckes langsam und so, daß im Inneren der Bereitschaftspackung Druckausgleich stattfinden kann, ohne daß dabei die Haube eingedrückt wird.

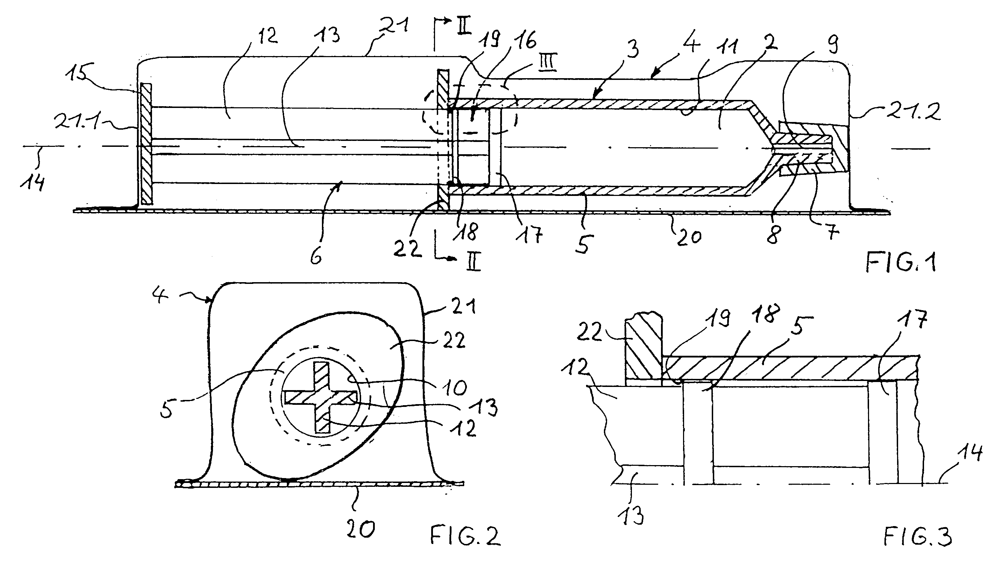

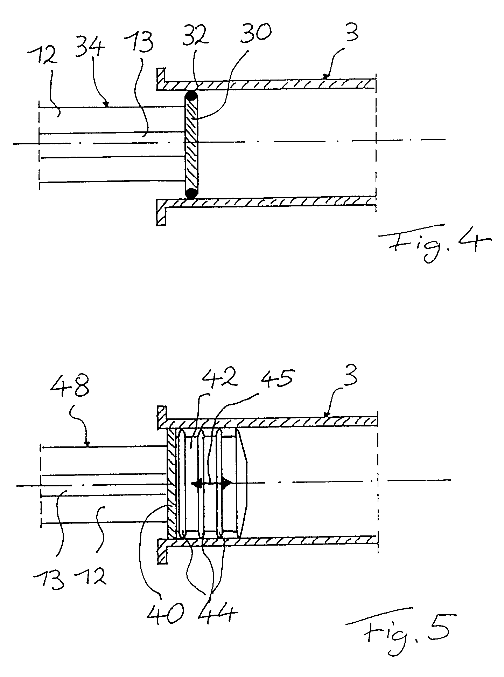

Bei dem in den Figuren 4 und 5 dargestellten bereichsweisen Längsschnitt geht es um die Abdichtung der Kolbenstange 6 innerhalb der Spritze 3. Gleiche Bauteile tragen dasselbe Bezugszeichen und werden nicht nochmals erläutert.

Bei der in Fig. 4 dargestellten Kolbenstange 34 ist eine Kolbenscheibe 30 vorhanden, die umfangsmäßig eine Nut aufweist, in der ein O-Dichtring 32 aus Gummi gelagert ist. Der O-Ring 32 gewährleistet auch nach dem Autoklavieren eine ausreichende Dichtigkeit trotz mitunter auftretendem Schrumpfverhalten der Kolbenstange selbst.

Die in Fig. 5 ausschnittsweise dargestellte Kolbenstange 48 weist eine Kreisscheibe 40 auf, an der stirnseitig ein Gummistopfen 42 als Dichteinrichtung angeordnet ist, der drei in Längsrichtung umlaufende elastische Dichtlamellen 44 aufweist.Der Gummistopfen ist in Längsrichtung (Pfeil 45) elastisch verformbar, so daß dadurch einem sich während des Sterilisierens im Inneren der Spritze 3 aufbauendem Druck zumindest teilweise entgegengewirkt werden kann.

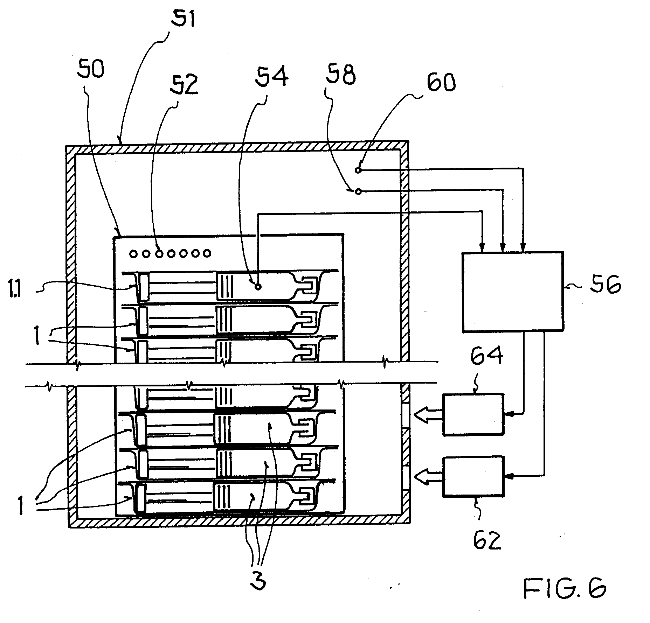

In Fig. 6 ist schematisch ein Autoklav 51 dargestellt, in den eine Verpackungseinheit 50 zum Autoklavieren eingebracht ist, welche Verpackungseinheit 50 mehrere Bereitschaftspackungen 1 aufweist, die mit einer gefüllten Spritze versehen sind. Die Verpackungseinheit 50 besteht aus zumindest einer Faltschachtel, die bereichsweise eine Perforation 52 aufweist, was in Fig. 6 schematisch dargestellt ist. Bei mindestens zwei Bereitschaftspackungen 1.1 in jeder Autoklavenladung ist im Inneren der Spritze 3 ein Temperatursensor 54 vorhanden, der die Temperatur des in den Spritzenkörper eingefüllten Arzneimittels während des Autoklaviervorgangs mißt. Dieser Temperatursensor 54 gibt ein Signal an eine Steuereinheit 56 ab, welche auf eine Heizeinrichtung und Kühleinrichtung 64 einwirkt und gegebenenfalls bei zu geringer Temperatur eine erhöhte Heizleistung bzw. nach der Sterilisation die geregelte Abkühlung der Autoklavenladung veranlaßt.

In einem speziellen Ausführungsbeispiel werden in einem Autoklav 51 eine Vielzahl (z.B. 1000) Faltschachteln 50, beschickt mit jeweils zehn Blistern 1, dampfsterilisiert. Dabei wird in zwei Spritzen 3, verteilt über das Sterilisationsgut (z.B. oben und in der Mitte), die Temperatur mit jeweils einem Meßfühler 54 erfaßt und kontrolliert (geregelt).Darüberhinaus sind im Autoklaven 51 ein Drucksensor 58 und ein Feuchtigkeitssensor 60 vorhanden, die ihre Signale ebenfalls an die Steuereinrichtung 56 abgeben. Die Steuereinrichtung 56 wirkt gegebenenfalls, d.h. in Abhängigkeit der Signale des Drucksensors 58 und des Feuchtigkeitssensors 60 auf eine Evakuiereinrichtung 62 ein, die eine Druckverminderung mit einer vorgegebenen Druckänderungsgeschwindigkeit ermöglicht. Der Feuchtigkeitsgehalt wird mit einem empirisch ermittelten Wert verglichen, welcher Wert danach ermittelt wird, daß möglichst wenig Kondensat anfällt, so daß soviel Feuchtigkeit im DampfLuftgemisch vorhanden ist, daß eine gute Wärmeübertragung auf das Füllgut im Behältnis zur raschen und einwandfreien Sterilisation erfolgt und andererseits möglichst wenig Kondensat anfällt, damit keine visuellen Beeinträchtigungen (Wasserflecken) am Blister und an der Faltschachtel auftreten oder Restfeuchte in der Packung verbleibt.

Bei dieser Verpackungseinheit 50 wird in einem Schritt die Bereitschaftspackung, die Außenseite der Spritze und die Füllung der Spritze sterilisiert. Ein aufwendiger aseptischer Füllvorgang kann somit entfallen. Darüberhinaus ermöglicht das erfindungsgemäße Verfahren, daß vielfach eingesetzte Konservierungsstoffe zur Haltbarmachung der Füllung nicht benötigt werden, da durch den Sterilisierungsvorgang eine ausreichend lange Lagerzeit gewährleistet werden kann, ohne Beeinträchtigung der Wirkung der Füllung. Dadurch können eventuell auftretende Allergiereaktionen aufgrund von Konservierungsstoffen vermieden werden..

So kann das erfindungsgemäße Verfahren bevorzugt auch für gefüllte Glasspritzen oder für andere medizinische Bedarfsartikel eingesetzt werden, bei denen eine neben der Sterilität der Füllung, d.h. des Arzneimittels selbst, auch eine Außensterilität gefordert wird, nämlich bevorzugt in den Bereichen einer Klinik, in denen derartige Bereitschaftspackungen intraoperativ, d.h. in Operationssälen bei Operationen am offenen Körper, benötigt bzw. eingesetzt werden.

## Patentansprüche

1. Verfahren zur Herstellung steriler Bereitschaftspackungen (1) oder einer Verpackungseinheit (50), die sterile Bereitschaftspackungen (1) enthält, bestehend aus
- zumindest einem eingesiegelten, mit einer Füllung (2) gefüllten Behältnis (3),
- bei dem die Packung (4) einen bevorzugt für Wasserdampfdiffusion durchlässigen Bereich (20) aufweist und im übrigen dicht ist,
- bei dem für das Behältnis (3) ein mindestens bei 125°C (Grad Celsius), vorzugsweise bei 130°C, beständiges Material eingesetzt wird und
- bei dem mehrere versiegelte, dicht verschlossene Packungen einem Autoklaviervorgang ausgesetzt werden,
**dadurch gekennzeichnet**, daß
- das Behältnis (3) mit einer nichtsterilen Füllung befüllt wird und danach in der Packung (4) eingesiegelt wird,
- in einem einzigen Arbeitsgang sowohl die Füllung (2) und die Außenoberflächen des Behältnisses (3) als auch die Packung (4) sterilisiert wird und
- zumindest in einer Bereitschaftspackung (1) die Temperatur der Füllung (2) durch einen Temperatursensor (54) gemessen wird und davon abhängig der Sterilisationsvorgang beim Autoklavieren geregelt wird, indem mindestens 15 Minuten, bevorzugt 20 Minuten, bei mindestens 121°C Temperatur der Füllung (2) mittels Dampf-Luftgemisch-Umwälzverfahren sterilisiert wird.

2. Verfahren nach Anspruch 1,
**dadurch gekennzeichnet**, daß
mehrere Bereitschaftspackungen (1) in einer Verpackungseinheit (50) bzw. mehrere Verpackungseinheiten (50) mit Bereitschaftspackungen (1) in einem Autoklaven angeordnet werden, welche einen für Wasserdampf durchlässigen Bereich (52) aufweist/aufweisen.

3. Verfahren nach Anspruch 1 und/oder 2,
**dadurch gekennzeichnet**, daß
während des gesamten Sterilisationszyklus der Druck und der Feuchtigkeitsgehalt gemessen werden und in Abhängigkeit der gemessenen Werte der Sterilisationszyklus geregelt wird, wobei der Druck bevorzugt mit einer vorgebbaren Druckänderungsgeschwindigkeit änderbar ist.

4. Verfahren nach Anspruch 2 und/oder 3,
**dadurch gekennzeichnet**, daß
für die Verpackungseinheit (50), bevorzugt Faltschachtel, Karton, bevorzugt zumindest bereichsweise perforierter Karton, eingesetzt wird.

5. Verfahren nach einem der vorhergehenden Ansprüche,
**dadurch gekennzeichnet**, daß
als Material für den Wasserdampfdiffusionsbereich der Bereitschaftspackung (1) Papier eingesetzt wird.

6. Verfahren nach einem der vorhergehenden Ansprüche zum Herstellen einer Bereitschaftspackung für Spritzen,
**dadurch gekennzeichnet**, daß
- eine leere mit einem Ausspritzzylinder (8) versehene Spritze (3) mit in Entleerungsstellung vorgeschobenem Kolben (6) bereitgehalten wird,
- in die bereitgehaltene Spritze (3) durch den Ausspritzzylinder (8) nichtsterile Füllung (2) dosiert eingedrückt wird und dadurch der Kolben (6) nach rückwärts zurückgedrücktwird,
- der Ausspritzzylinder der gefüllten Spritze (3) mit einer Verschlußkappe (7) aus zumindest bei 121 ^{o}C beständigem elastischen Material verschlossen wird,
- die gefüllte, verschlossene Spritze (3) in eine Einsiegelungspackung (1) , insbesondere einen Blister, eingesetzt wird,
- die Einsiegelungspackung (1) verschlossen und versiegelt wird,
- eine mehrere so erhaltene Bereitschaftspackungen (1) aufweisende Verpackungseinheit (50) im Autoklav (51), vorzugsweise nach vorheriger Evakuierung, im Wasserdampf auf 121 ^{o}C durchgewärmt und über mindestens 20 Minuten auf 121 ^{o}C durchgewärmt gehalten wird,
- die Bereitschaftspackung (1) dann unter Einführung eines Stützdrucks abgekühlt und dabei dekompressiert wird und
- die Verpackungseinheit (50) mit der Bereitschaftspackung (1) unter Anwendung von einem oder mehreren Evakuierungszyklen im Autoklaven vollständig getrocknet wird.

7. Verfahren nach einem oder mehreren der Ansprüche 1 bis 4 zum Herstellen einer Bereitschaftpackung für Spritzen,
**dadurch gekennzeichnet,** daß
- eine Spritze (3) ohne Kolbenstange (6) bereitgehalten wird, deren Ausspritzzylinder (8) mit einer Verschlußkappe (7) oder einer Injektionskanüle mit Nadelschutzkappe verschlossen ist, die aus zumindest bei 121 ^{o}C beständigem elastischen Material besteht,
- in die bereitgehaltene Spritze (3) von der Öffnung (10) für die Kolbenstange (6) her dosiert nichtsterilé Füllung (2) eingebracht wird,
- in die gefüllte Spritze (3) die Kolbenstange (6) dichtend eingeführt wird,
- die gefüllte, verschlossene Spritze (3) in eine Einsiegelungspackung, insbesondere einen Blister, eingesetzt wird,
- die Einsiegelungspackung nun versiegelt wird,
- eine mehrere Bereitschaftspackungen (1) aufweisende Verpackungseinheit (50) im Autoklav (51), vorzugsweise nach vorheriger Evakuierung, im Wasserdampf auf 121 ^{o}C durchgewärmt und über mindestens 20 Minuten auf 121 ^{o}C durchgewärmt gehalten wird und
- die Bereitschaftspackungen (1) dann langsam, vorzugsweise unter Einführung eines Stützdrucks, abgekühlt und dabei dekompressiert wird.

8. Bereitschaftspackung (1) mit einer mit einer Füllung versehenen Spritze (3) als Behältnis zur Ausübung des Verfahrens nach einem der vorhergehenden Ansprüche, wobei
- ein Spritzenkörper (5) einen kreiszylindrischen, nach hinten auf seiner ganzen Querschnittsfläche offenen und nach vorn in einen Ausspritzzylinder (8) mündenden mindestens umhüllten Füllraum (11) aufweist,
- eine langgestreckte Kolbenstange (6) koaxial zum Füllraum angeordnet ist, die mit ihrem hinteren Ende aus dem Füllraum herausragt, an ihrem hinteren Ende eine Handhabe (15) und an ihrem vorderen Ende einen Kolben (16) aufweist, der längsverschieblich in den Füllraum paßt,
- der Kolben zumindest eine, stirnseitig an der Kolbenstange angeordnete, dichtend in den Füllraum passende Kolbenscheibe (17) aufweist,
- eine auf den Ausspritzzylinder (8) aufsetzbare, bevorzugt entlang des Ausspritzkanals (9) verschiebbare Verschlußkappe (7) vorgesehen ist und
- alle Teile aus mindestens bei 121 ^{o}C beständigem Material bestehen
**dadurch gekennzeichnet**, daß
- die Packung (4) einen für Wasserdampfdiffusion durchlässigen Breich aufweist und im übrigen dicht ist.

9. Packung nach Anspruch 8,
**dadurch gekennzeichnet,** daß
bei der Spritze stirnseitig vor der Kolbenscheibe (40) eine in Kolbenrichtung elastisch verformbare Dichteinrichtung (42), insbesondere aus Gummi, mit zumindest einer umfangsmäßig umlaufenden Dichtlamelle (44) vorhanden ist.

10. Verpackungseinheit (50) mit zumindest einer Bereitschaftspackung (1) aus einem verblisterten, mit einem Arzneimittel gefüllten Behältnis, herstellbar nach einem der Ansprüche 1 bis 7,
**dadurch gekennzeichnet,** daß
- die Packung (4) eine Haube (21) mit stirnseitigen Wandbereichen (21.1, 21.2) besitzt und
- das Behältnis so in der Packung (4), bevorzugt dem Blister, angeordnet wird, daß die entsprechenden Stirnseiten des Behältnisses (3) an den Wandbereichen (21.1, 21.2) anliegen, so daß zeitweise auftretende Längenausdehnungen des Behältnisses infolge des Sterilisations(Erwärmungs)vorgangs durch die Wandung des Behältnisses zumindest teilweise unterdrückt werden.

## Claims

1. A method for fabricating a sterile ready-pack (1) or a packing unit (50) which contains sterile ready-packs, comprising
- at least one sealed-in container (3) filled with a fill (2),
- such that the packing (4) has a region (20) that is permeable to the diffusion of steam and for the rest is tight,
- such that a material that is stable at least at 125°C (degrees Celsius), preferably at 130°C is used for the container (3), and
- such that several sealed, tightly closed packages are exposed to an autoclaving process,
characterized in that
- the container (3) is filled with a non-sterile fill and then is sealed into the package (4),
- in a single operating step, both the fill (2) and the external surfaces of the container (3) as well as the packing (4) are sterilized, and
- the temperature of the fill (2) is measured at least in one ready-pack (1) by a temperature sensor (54), and the sterilization process during autoclaving is regulated in dependence on this, such that the fill (2) is sterilized by means of a steam-air mixture, in a rotary process, for at least 15 minutes, preferably 20 minutes, at least at 121°C.

2. The method of Claim 1,
characterized in that
several ready-packs (1) in one packing unit (50) or several packing units (50) with ready-packs (1) are disposed in an autoclave, which have a region (52) which is permeable to steam.

3. The method of Claim 1 and/or 2,
characterized in that
the pressure and moisture are measured during the entire sterilization cycle, and the sterilization cycle is regulated in dependence on the measured values, such that the pressure can be varied preferably at a specifiable rate.

4. The method of Claim 2 and/or 3,
characterized in that
preferably folding boxes, cardboard, cardboard that is perforated at least in certain parts, is used for the packing unit (50).

5. The method of one of the preceding claims,
characterized in that
paper is used as the material for the steam diffusion area of the ready pack (1).

6. The method of one of the preceding claims, for fabricating a ready-pack for syringes,
characterized in that
- an empty syringe (3), equipped with a discharge cylinder (8), with a piston (6) advanced into the discharge position, is held ready,
- the non-sterile fill (2) is dosed and pressed into the ready syringe (3) through the discharge cylinder (8), and thus the piston (6) is pressed backward,
- the discharge cylinder of the filled syringe (3) is closed with a closure cap (7) consisting of an elastic material that is stable at least at 121°C,
- the filled, closed syringe (3) is inserted into a sealing pack (1), especially a blister,
- the sealing pack (1) is closed and sealed,
- a packing unit (50) containing one or more such resulting ready-packs (1) is heated in the autoclave (51) to 121°C by steam, preferably after previous evacuation, and is kept heated at 121°C for at least 20 minutes,
- the ready-pack (1) is then cooled while a support pressure is introduced, and is thus decompressed, and
- the packing unit (50) with the ready-pack (1) is completely dried in the autoclave by using one or more evacuation cycles.

7. The method of one or more of Claims 1 to 4 for fabricating a ready-pack for syringes,
characterized in that
- a syringe (3) without a piston rod (6) is kept ready, whose discharge cylinder (8) is closed by means of a closure cap (7) or an [sic, probably should be "whose"] injection needle is closed with a needle protection cap, which consists of an elastic material that is stable at least at 121°C,
- the fill (2) is dosed and introduced into the ready syringe (3) from the opening (10) for the piston rod (6)
- the piston rod (6) is sealingly introduced into the filled syringe (3),
- the filled, closed syringe (3) is inserted into a sealing pack, especially a blister,
- the sealing pack is now sealed,
- a packing unit (50) containing one or more ready-packs (1) is heated to 121°C by steam in the autoclave (51), preferably after previous evacuation, and is kept heated at 121°C for at least 20 minutes, and
- the ready-pack (1) is then slowly cooled, preferably while a support pressure is introduced, and thus is decompressed.

8. A ready-pack (1), with a syringe (3) containig a fill, as a container for performing the method of one of the preceding claims, such that
- a syringe body (5) has a cylindrical fill space (11), at least enclosed, open toward the rear along its entire cross-sectional area and in the front going over into a discharge cylinder (8),
- a longitudinally extended piston rod (6) is disposed coaxial to the fill space, the rear end of said piston rod (6) extending out of the fill space and having a handle (15), the front end of said piston rod (6) having a piston (16) which fits into the fill space so that it can be moved longitudinally,
- the piston has at least one piston disk (17), disposed frontally at the piston rod and fitting sealingly in the fill space,
- a closure cap (7) is provided, which can be placed on the discharge cylinder (8), and which preferably can be moved along the discharge channel (9), and
- all parts consist of a material that is stable at least at 121°C, characterized in that
- the pack (4) has a region that is permeable to the diffusion of steam and for the rest is tight.

9. The pack of Claim 8,
characterized in that
a sealing device (42), which can be elastically deformed in the piston direction, and which consists especially of rubber, with at least one circumferential sealing lamella (44), is present at the syringe frontally before the piston disk (40).

10. A packing unit (50) consisting of at least one ready-pack with a blister-type container filled with a medication, fabricated in accordance with one of the Claims 1 to 7,
characterized in that
- the pack (4) has a hood (29) with frontal wall areas (21.1, 21.2) and
- the container is disposed in the ready-pack, preferably the blister, such that the corresponding face sides of the container (3) abut the wall areas (21.1, 21.2), so that longitudinal expansions of the container, which may sometimes occur due to the sterilization (heating) process, through the wall of the container, are at least partially suppressed.

## Revendications

1. Procédé de fabrication de conditionnements (1) stériles de produits prêts à l'emploi, ou d'un emballage de groupage (50) qui contient des conditionnements (1) stériles de produits prêts à l'emploi, comprenant chacun
- au moins un contenant (3) sous l'emballage scellé et rempli d'une charge (2),
- selon lequel l'emballage (4) présente une zone perméable (20), de préférence à la diffusion de vapeur d'eau, et est par ailleurs étanche,
- selon lequel un matériau stable à au moins 125° C (degrés Celsius), de préférence à 130° C, est utilisé pour le contenant (3) et
- selon lequel plusieurs emballages scellés, fermés étanches, sont exposés à un autoclavage,
caractérisé en ce que
- on remplit le contenant (3) avec une charge non stérile et on le scelle ensuite dans l'emballage (4),
- on stérilise à la fois la charge (2) et les surfaces extérieures du contenant (3) et l'emballage (4) en une seule opération et
- on mesure la température de la charge (2) dans au moins un conditionnement (1) de produit prêt à l'emploi par un capteur de température (54) et on règle, en fonction de la température ainsi mesurée, l'opération de stérilisation lors d'un autoclavage au cours duquel la stérilisation est effectuée au moyen d'un processus de brassage d'un mélange de vapeur et d'air pendant au moins 15 minutes, de préférence pendant 20 minutes, à une température de la charge (2) d'au moins 121° C.

2. Procédé selon la revendication 1, caractérisé en ce que l'on traite dans un autoclave plusieurs conditionnements (1) de produits prêts à l'emploi contenus dans un emballage de groupage (50), ou plusieurs emballages de groupage (50) contenant des conditionnements (1) de produits prêts à l'emploi, l'emballage de groupage ou chaque emballage de groupage présentant une zone (52) perméable à la vapeur d'eau.

3. Procédé selon la revendication 1 et/ou 2, caractérisé en ce que, pendant tout le cycle de stérilisation, on mesure la pression et la teneur en humidité et on règle le cycle de stérilisation en fonction des valeurs mesurées, la pression étant de préférence variable à une vitesse de variation de pression pouvant être préfixée.

4. Procédé selon la revendication 2 et/ou 3, caractérisé en ce que l'on utilise du carton, de préférence du carton au moins perforé par endroits, pour l'emballage de groupage (50), se présentant de préférence sous la forme d'une boîte pliante.

5. Procédé selon une des revendications précédentes, caractérisé en ce que l'on utilise du papier comme matériau pour la zone perméable à la diffusion de vapeur d'eau du conditionnement (1) de produit prêt à l'emploi.

6. Procédé selon une des revendications précédentes appliqué à la fabrication d'un conditionnement de produit prêt à l'emploi sous la forme d'une seringue, caractérisé en ce que
- on maintient prête une seringue (3) vide, pourvue d'un embout cylindrique d'éjection (8), avec le piston (6) de la seringue avancé à une position de vidage,
- on introduit sous pression et de façon dosée une charge (2) non stérile à travers l'embout cylindrique d'éjection (8), en repoussant ainsi le piston (6) vers l'arrière,
- on ferme l'embout cylindrique d'éjection de la seringue (3) remplie par un capuchon de fermeture (7) fabriqué d'un matériau élastique stable à au moins 121° C,
- on place la seringue (3) remplie et fermée dans un emballage de scellement (1), en particulier un blister,
- on ferme et on scelle l'emballage de scellement (1),
- on chauffe à coeur un emballage de groupage (50) contenant plusieurs conditionnements (1) de produits prêts à l'emploi ainsi obtenus dans un autoclave (51), de préférence évacué préalablement, le chauffage à coeur s'effectuant à la vapeur d'eau jusqu'à 121° C et étant maintenu pendant au moins 20 minutes à 121° C,
- on refroidit en le décompressant le conditionnement (1) de produit prêt à l'emploi en introduisant une pression de soutien et
- on sèche complètement l'emballage de groupage (50) avec les conditionnements (1) de produits prêts à l'emploi en appliquant un ou plusieurs cycles d'évacuation dans l'autoclave.

7. Procédé selon une ou plusieurs des revendications appliqué à la fabrication d'un conditionnement de produit prêt à l'emploi sous la forme d'une seringue,
caractérisé en ce que
- on maintient prête une seringue (3) sans tige de piston (6) et dont un embout cylindrique d'éjection (8) est fermé par un capuchon (7) ou une canule d'injection pourvue d'un capuchon protecteur d'aiguille fait d'un matériau élastique stable à au moins 121° C,
- on introduit de façon dosée une charge non stérile dans la seringue (3) maintenue prête par l'ouverture (10) pour la tige de piston (6),
- on engage la tige de piston (6) de façon étanche dans la seringue (3) remplie,
- on place la serinfue (3) remplie et fermée dans un emballage de scellement, en particulier un blister,
- on scelle l'emballage de scellement;
- on chauffe à coeur un emballage de groupage (50) contenant plusieurs conditionnements (1) de produits prêts à l'emploi dans un autoclave (51), de préférence évacué préalablement, le chauffage à coeur s'effectuant à la vapeur d'eau jusqu'à 121° C et étant maintenu pendant au moins 20 minutes à 121° C et
- on refroidit ensuite lentement les conditionnements (1) de produits prêts à l'emploi, de préférence en introduisant une pression de soutien et en les décompressant.

8. Conditionnement (1) de produit prêt à l'emploi, comprenant une seringue (3), pourvue d'une charge, en tant que contenant pour la mise en oeuvre du procédé selon une des revendications précédentes, conditionnement dans lequel
- un corps de seringue (5) présente et entoure au moins un espace de remplissage (11) ayant la forme d'un cylindre circulaire qui est ouvert à l'arrière sur la totalité de son aire de section droite, débouche à l'avant dans un embout cylindrique d'éjection (8), et est au moins entoure,
- une tige de piston (6) de forme allongée est disposée dans l'espace de remplissage, coaxialement par rapport à celui-ci, et dépasse par son extrémité arrière de cet espace, extrémité où elle porte un élément de manoeuvre (15), tandis que son extrémité avant porte un piston (16) ajusté longitudinalement coulissant dans l'espace de remplissage,
- le piston présente au moins un disque (17) placé à l'extrémité de la tige de piston et ajusté de façon étanche dans l'espace de remplissage,
- un capuchon de fermeture (7), pouvant être mis en place sur l'embout cylindrique d'éjection (8), est disposé de préférence coulissant le long du canal d'éjection (9) délimité par cet embout et
- toutes les pièces sont faites d'un matériau stable à au moins 121° C,
caractérisé en ce que l'emballage (4) présente une zone perméable à la diffusion de vapeur d'eau et est étanche par ailleurs.

9. Conditionnement selon la revendication 8, caractérisé en ce que la seringue comporte, devant la face de tête du disque de piston (40), un dispositif d'étanchéité (42) élastiquement déformable en direction du piston, en particulier en caoutchouc, sur lequel au moins une lamelle d'étanchéité (44) fait tout le tour du bouchon.

10. Emballage de groupage (50) contenant au moins un conditionnement (1) de produit prêt à l'emploi, comprenant un contenant blistérisé et rempli d'un médicament, qui peut être fabriqué selon une des revendications 1 à 7,
caractérisé en ce que
- l'emballage (4) possède une coque (21) avec des zones de paroi extrêmes (21.1, 21.2) et
- le contenant est placé dans l'emballage (4), formé de préférence par un blister, de manière que les côtés extrêmes correspondants du contenant (3) s'appliquent contre les zones de paroi extrêmes (21.1, 21.2), de sorte que des dilatations longitudinales du contenant, se produisant temporairement par suite de l'opération de stérilisation (de chauffage) soient au moins partiellement supprimées par la paroi du contenant.
